# NEUE EUROPÄISCHE PATENTSCHRIFT

(11) **EP 2 571 451 B2**
(45) Veröffentlichungstag und Bekanntmachung des Hinweises auf die Entscheidung über den Einspruch: **17.03.2021**
(45) Hinweis auf die Patenterteilung: 26.03.2014
(21) Anmeldenummer: 12721695.0
(22) Anmeldetag: 18.04.2012
(51) Int. Cl.: A61C 13/00, A61C 13/12, A61C 19/10, A61C 13/10

(54) **SET AUS PROTHESENZAHNTRÄGER UND PROTHESENZAHN, VERFAHREN ZUR BEARBEITUNG EINES PROTHESENZAHNS UND VERFAHREN ZUR HERSTELLUNG DES SETS**
SET CONSISTING OF A PROSTHETIC TOOTH SUPPORT AND A PROSTHETIC TOOTH, METHOD OF PROCESSING A PROSTHETIC TOOTH AND METHOD OF MAKING THE SET
ENSEMBLE D'UN SUPPORT DE DENT PROTHÉTIQUE COMPORTANT UNE DENT PROTHÉTIQUE, PROCÉDÉ DE TRAITEMENT D'UNE DENT PROTHÉTIQUE
ET PROCÉDÉ DE PRODUCTION DE L'ENSEMBLE

(30) Priorität: 16.05.2011 DE 102011101678
(43) Veröffentlichungstag der Anmeldung: 27.03.2013
(60) Teilanmeldung: 13004153.6
(73) Patentinhaber: Amann Girrbach AG, 6842 Koblach (AT)
(72) Erfinder: NOACK, Falko, A-6890 Lustenau (AT)
(74) Vertreter: Hofmann, Ralf U.
(86) Internationale Anmeldenummer: PCT/AT2012/000105
(87) Internationale Veröffentlichungsnummer: WO 2012/155161

(56) Entgegenhaltungen:
- EP-A1- 1 243 233
- EP-A1- 1 304 089
- WO-A1-95/30382
- DE-A1- 19 508 760
- DE-A1- 19 532 171
- DE-A1- 19 532 171
- US-A- 6 079 981
- US-A1- 2002 125 619
- US-A1- 2007 190 492

## Beschreibung

Die vorliegende Erfindung betrifft ein Set gemäß des Oberbegriffs des Patentanspruchs 1. Weiters betrifft die Erfindung auch ein Verfahren zur Bearbeitung zumindest eines Prothesenzahns eines solchen Sets und Verfahren zur Herstellung eines solchen Sets.

Dentale Voll- oder Teilprothesen bestehen grundsätzlich aus zumindest zwei unterschiedlichen Komponenten. Dies ist zum Einen eine Prothesenbasis, welche die fehlenden Zahnfleischanteile ersetzt. Zum Anderen sind dies die Prothesenzähne bzw. Ersatzzähne, welche als Ersatz für die fehlenden natürlichen Zähne des Patienten fungieren. Die Prothesenzähne sind mit der Prothesenbasis verbunden und haben sowohl therapeutische als auch ästhetische Funktionen. Prothesenzähne werden von verschiedenen Herstellern angeboten. Alle am Markt erhältlichen Prothesenzähne sind konfektionierte Formen, die in ihren Farben variieren. Es existieren am Markt in verschiedenen Größen vorkonfektionierte Zahnformen, welche vom Zahntechniker entsprechend bestimmter individueller Charakteristika des Patienten wie z.B. Platzangebot, Körpergröße, Geschlecht und Figur ausgewählt werden. Die am Markt erhältlichen Prothesenzähne werden für die Anwendung in Voll- und Teilprothesen im Patientengebiss weitestgehend unbearbeitet verwendet. Hierzu bleibt speziell der Kronenbereich der Prothesenzähne weitgehend unversehrt. Der Hals- bzw. Wurzelbereich der Prothesenzähne muss jedoch in den meisten Fällen auf Grund der unterschiedlichen Bisshöhen, also dem unterschiedlichen Abstand zwischen Ober- und Unterkiefer der einzelnen Patienten angepasst werden. Hierzu werden die Zahnhals- und Wurzelbereiche der Prothesenzähne eingekürzt. Dies wird beim Stand der Technik vom Zahntechniker frei Hand realisiert, wodurch einerseits Ungenauigkeiten, andererseits aber auch ein erheblicher Zeitaufwand entstehen, da die Prothesenzahnlänge immer wieder überprüft werden muss. Beim Einkürzen der Zähne versucht man möglichst viele Anteile des Zahnhalses unbearbeitet zu lassen und nur so wenig wie möglich einzukürzen bzw. abzutragen. Der Zahnhals- und Wurzelbereich des Prothesenzahnes dient als Verbindungsfläche zur Prothesenbasis, hat aber auch wichtige Funktionen für die Ästhetik des Prothesenzahns und damit der gesamten Prothese.

In der gesamten Zahntechnik hat in den letzten Jahren immer mehr die automatisierte bzw. rechnergesteuerte Herstellung von Zahnersatz Einzug gehalten. Insbesondere die Herstellung von Vollprothesen stellt aktuell ein Betätigungsfeld im Bereich der dentalen CAD-CAM Entwicklung dar. Dies betrifft sowohl das Design der Prothesenbasis als auch die Positionierung der einzelnen Prothesenzähne relativ zur Prothesenbasis. Aus diesem Grund werden für die am Markt erhältlichen Prothesenzähne auch bereits die Informationen über deren Form digitalisiert, also als Datensätze angeboten. Um die digital bzw. virtuell zusammengesetzte bzw. designte Vollprothese nun aber auch reell umsetzen zu können, müssen nach wie vor die am Markt erhältlichen Prothesenzähne eingekürzt werden, um in der Prothesenbasis entsprechend Platz zu finden.

Beim Stand der Technik werden die Prothesenzähne auf Prothesenzahnträgern am Markt angeboten, wobei die Prothesenzähne allerdings nur über eine Haftmasse leicht haftend auf den Prothesenzahnträger aufgeklebt sind. Die hierfür verwendeten Haftmittel dienen lediglich dazu, sicher zu stellen, dass die Prothesenzähne beim Transport nicht verloren gehen und zur besseren Ansicht positioniert sind. Die Haftmittel sind so schwach, dass die Prothesenzähne ohne Weiteres händisch vom Prothesenzahnträger entfernt werden können.

EP-A-1 243 233 beschreibt einen Prothesenzahnträger mit mehreren Prothesenzähnen. Der Kronenbereich der Prothesenzähne ist bereichsweise in eine Trägerschicht eingebettet. Am Prothesenzahnträger ist eine Kodierung in Form eines Strichcodes angebracht, welcher die Charge des Herstellers identifiziert.

DE-A-195 32 171 beschreibt ein Verfahren zum Herstellen einer Zahnprothese, bei der unter Berücksichtigung der anatomischen Begebenheiten des Patienten die optimale Position der Prothesenzähne als Datensatz vorliegen und die Prothesezähne von einer Montagevorrichtung entsprechend auf eine Montageplatte gesetzt werden.

Die US 2007/0190492 A1 offenbart gattungsgemäßen Stand der Technik.

Es ergibt sich die Aufgabe, Prothesenzähne für einen automatisierten Bearbeitungsschritt in einer alternativen Art und Weise sicher in an sich bekannten, mittels Datenverarbeitungseinrichtungen gesteuerten Fräseinrichtungen in der Weise positionieren zu können, dass die Zahnhals- und Wurzelbereiche der Prothesenzähne automatisiert in der gewünschten Art und Weise eingekürzt werden können.

Zur Lösung dieser Aufgabe wird ein Set gemäß des Patentanspruchs 1 vorgeschlagen.

Es ist somit eine Grundidee der Erfindung, den Prothesenzahn oder die Prothesenzähne jeweils mit ihrem Kronenbereich so in einer Trägerschicht des Prothesenzahnträgers einzugießen oder zu umspritzen, dass der Zahnhals- und Wurzelbereich der Prothesenzähne aus der Trägerschicht herausschaut und mittels geeigneter, beim Stand der Technik z.B. in Form von CNC-Fräsmaschinen bekannter Fräseinrichtungen bearbeitet bzw. gekürzt werden kann. Dies ermöglicht es, die Prothesenzähne in definierten Positionen in der Trägerschicht einzugießen oder zu umspritzen. Die definierten Positionen im Prothesenzahnträger und die digital bereits vorhandene oder gegebenenfalls durch Einscannen oder andere geeignete Mittel zu erstellende Information über die Zahnform können dann in dem, vorzugsweise auf einem einheitlichen Koordinatensystem basierenden, Datensatz abgespeichert und einer, die Fräseinrichtung steuernden Datenverarbeitungseinrichtung zugeführt werden.

Um einen sicheren Halt während der Bearbeitung zu gewährleisten, bietet es sich an, die Prothesenzähne in der Trägerschicht in einem entsprechend festen aber fräsbaren Material, beispielsweise Wachs oder Kunststoff, welches keine chemische Verbindung mit dem Prothesenzahn eingeht, einzugießen oder zu umspritzen. Da die Trägerschicht die Funktion des In-Positionhaltens der Prothesenzähne vollständig ausfüllt, kann in einer Variante der Erfindung vorgesehen sein, dass ein Zahnhals- und Wurzelbereich des Prothesenzahnes bzw. der Prothesenzähne zumindest bereichsweise, vorzugsweise vollständig, frei einsehbar über die Trägerschicht übersteht. Der Kronenbereich des Prothesenzahnes bzw. der Prothesenzähne ist zumindest bereichsweise in die Trägerschicht mittels Eingießen oder Umspritzen eingebettet. Er kann auch vollständig in die Trägerschicht mittels Eingießen oder Umspritzen eingebettet sein.

Zur Herstellung eines solchen Sets sieht ein bevorzugtes Verfahren vor, dass der Prothesenzahn bzw. die Prothesenzähne mit dem Zahnhals- und Wurzelbereich in einer Hilfshaltevorrichtung positioniert wird bzw. werden und dann der Kronenbereich des Prothesenzahns bzw. der Prothesenzähne zumindest bereichsweise, vorzugsweise vollständig, in der Trägerschicht mittels Eingießen oder Umspritzen eingebettet wird bzw. werden.

Um die Prothesenzähne nach dem Einkürzen des Zahnhals- und Wurzelbereichs aus der Trägerschicht entnehmen zu können, weist diese günstigerweise eine Sollbruchstelle auf. Diese Sollbruchstelle kann bereits herstellerseitig oder vom Anwender mittels Fräseinrichtung in die Trägerschicht eingebracht werden.

Es muss aber nicht zwingend vorgesehen sein, dass die Zahnhals- und Wurzelbereiche des Prothesenzahnes bzw. der Prothesenzähne frei einsehbar sind. Alternativ kann auch vorgesehen sein, dass ein Zahnhals- und Wurzelbereich des Prothesenzahnes bzw. der Prothesenzähne zumindest bereichsweise, vorzugsweise vollständig, in eine Abdeckschicht des Prothesenzahnträgers eingebettet, vorzugsweise eingegossen, ist. Bei dieser Variante können der oder die Prothesenzähne also vollständig im Prothesenzahnträger verborgen sein. Dies stört die Bearbeitung bzw. das Einkürzen nicht, da die Positionen und die Formen der Prothesenzähne ja über den bereits genannten Datensatz bekannt sind.

Ein bevorzugtes erfindungsgemäßes Verfahren zur Herstellung eines Sets, bei dem die Zahnhals- und Wurzelbereiche in der Abdeckschicht eingebettet sind, sieht vor, dass der Prothesenzahn bzw. die Prothesenzähne mit dem Kronenbereich in einer Hilfshaltevorrichtung positioniert wird bzw. werden und dann der Zahnhals- und Wurzelbereich des Prothesenzahnes bzw. der Prothesenzähne zumindest bereichsweise, vorzugsweise vollständig, in der Abdeckschicht des Prothesenzahnträgers eingebettet, vorzugsweise eingegossen, wird bzw. werden und daran anschließend der Kronenbereich des Prothesenzahns bzw. der Prothesenzähne zumindest bereichsweise, vorzugsweise vollständig, in die Trägerschicht mittels Eingießen oder Umspritzen eingebettet wird bzw. werden.

Um den Prothesenzahnträger sicher und in einer definierten Positionierung in oder an der Fräseinrichtung befestigen zu können, sieht die Erfindung vor, dass an der Trägerschicht ein, gegebenenfalls zapfenförmig von der Trägerschicht abstehender, Fräsmaschinenadapter des Prothesenzahnträgers zur Befestigung des Prothesenzahnträgers während eines Fräsvorgangs in einer Fräseinrichtung befestigt ist. Im Sinne einer Vermeidung von Bedienungsfehlern weist der Fräsmaschinenadapter günstigerweise formschlüssige Indexierungen z.B. in seiner Oberfläche auf, damit er nur in einer einzigen bzw. eindeutigen Position in der Fräsmaschine befestigt werden kann. Solche Indexierungen sind an sich bekannt und können in Form von Kerben, Anschlägen, Polygonzügen und dergleichen ausgebildet sein.

Die Abdeckschicht sollte, sofern sie vorhanden ist, aus einem fräsbaren Material bestehen. Damit die fertig eingekürzten Prothesenzähne dann aus der Trägerschicht auch entfernt werden können, besteht diese günstigerweise ebenfalls aus einem fräsbaren Material. Grundsätzlich kommen dabei verschiedene Kunststoffe oder Wachse als solche Materialien in Frage. Im Sinne der Fräsbarkeit ist jedenfalls günstigerweise vorgesehen, dass die Trägerschicht und/oder die Abdeckschicht ein Material aufweist bzw. aufweisen oder daraus besteht bzw. daraus bestehen, welches eine Vickershärte zwischen 10 HV und 200 HV aufweist. Günstige Ausgestaltungsformen der Erfindung sehen jedenfalls vor, dass das Material der Trägerschicht zumindest so hart wie oder härter als das Material der Abdeckschicht ist.

Mit einem oben bereits genannten erfindungsgemäßen Set aus einem erfindungsgemäßen Prothesenzahnträger und dem zugeordneten Datensatz sieht ein erfindungsgemäßes Verfahren zur Bearbeitung zumindest eines Prothesenzahnes, vorzugsweise mehrerer Prothesenzähne, eines erfindungsgemäßen Sets vor, dass der Prothesenzahnträger des Sets mittels seines Fräsmaschinenadapters in eine Fräseinrichtung eingespannt wird und der Datensatz des Sets in eine Datenverarbeitungseinrichtung eingelesen wird und die Fräseinrichtung von der Datenverarbeitungseinrichtung zum Bearbeiten des Prothesenzahns oder der Prothesenzähne unter Berücksichtigung des Datensatzes angesteuert wird. Hierdurch wird es möglich, computergesteuert die einzukürzenden Bereiche des Zahnhals- und Wurzelbereichs der Prothesenzähne im Prothesenzahnträger gezielt anzufahren und auszufräsen. Dies ermöglicht es, am Computer durchgeführte digitale bzw. virtuelle Modelle einer dentalen Teil- oder Vollprothese in die reale Welt umzusetzen, indem hierfür die Prothesenzähne in der benötigten Art und Weise eingekürzt werden.

Zudem ist es möglich, auch zusätzliche Nuten oder Rillen in dem Prothesenzahn während des beschriebenen Frässchnittes einzuarbeiten, welche danach als Rotationsschutz oder zusätzliche Retention für den Prothesenzahn in der Prothesenbasis fungieren.

Sollte während der Konstruktion der Prothese ein Einkürzen der Prothesenzähne auch im Kronenbereich als notwendig ermittelt worden sein, so ist auch diese Manipulation der Zähne während des Fräsvorganges möglich, zumal die Trägerschicht, wie oben bereits ausgeführt, günstigerweise aus einem fräsbaren Material besteht.

Im Sinne einer besonders hohen Bedienerfreundlichkeit kann bei einem solchen Verfahren vorgesehen sein, dass der Datensatz im oder am Prothesenzahnträger, vorzugsweise im oder am Fräsmaschinenadapter, gespeichert wird und vor dem oder beim oder nach dem Einspannen des Prothesenzahnträgers in die Fräseinrichtung, vorzugsweise automatisch, von der Datenverarbeitungseinrichtung eingelesen wird. Der Prothesenzahnträger kann also selbst einen Datenträger wie z.B. einen Transponder, Balkencode oder dergleichen aufweisen, auf welchem der Datensatz abgespeichert ist. Dieser Datenträger kann am Fräsmaschinenadapter oder an anderer geeigneter Stelle am Prothesenzahnträger angeordnet sein. Die Datenverarbeitungseinrichtung kann dann den Datensatz automatisch oder nach Abfrage einlesen, sobald der Prothesenzahnträger in die Fräseinrichtung eingespannt ist.

Der Vollständigkeit halber sei darauf hingewiesen, dass der Datensatz des genannten erfindungsgemäßen Sets natürlich nicht zwingend am Prothesenzahnträger direkt angeordnet sein muss. Der Datensatz kann auch anderweitig gespeichert und in die Datenverarbeitungseinrichtung eingelesen werden. Hierzu stehen beim Stand der Technik zahlreiche, an sich bekannte Varianten zur Verfügung, welche hier nicht alle im Einzelnen aufgelistet werden müssen.

Der Vollständigkeit halber sei darauf hingewiesen, dass unter einem Prothesenzahn ein künstlicher Zahn zu verstehen ist, welcher in eine dentale Voll- oder Teilprothese eingebaut werden soll. Der Kronenbereich umfasst den äußeren Teilbereich des Prothesenzahnes, welcher bei einem gesunden natürlichen Zahn aus dem Zahnfleisch herausschaut. Der Kronenbereich umfasst die Kau- bzw. Okklusalfläche des Zahnes. Der Zahnhals- und Wurzelbereich eines natürlichen Zahnes ist bei gesundem Zahnfleisch in diesem verborgen. Im Falle eines Prothesenzahnes ist der Zahnhals- und Wurzelbereich bei fertiggestellter Teil- oder Vollprothese zumindest großteils in der Prothesenbasis verborgen. Der Zahnhals- und Wurzelbereich umfasst die Basalfläche des Prothesenzahns und dient der Befestigung des Prothesenzahns in einer entsprechenden Ausnehmung in der Prothesenbasis.

Weiters sei noch darauf hingewiesen, dass geeignete Fräseinrichtungen und Datenverarbeitungseinrichtungen beim Stand der Technik in Form von verschiedensten CAD- und CNC-Fräseinrichtungen mit entsprechender Rechneransteuerung bekannt sind und hier nicht weiter erläutert werden müssen.

Weitere Merkmale und Einzelheiten bevorzugter Ausgestaltungsformen der Erfindung werden anhand der nachfolgenden Figurenbeschreibung erläutert. Es zeigen:
- Fig. 1 und 2: beispielhaft beim Stand der Technik an sich bekannte Prothesenzähne;
- Fig. 3: die beim Stand der Technik an sich bekannte Anordnung dieser Prothesenzähne in einer im Querschnitt dargestellten Vollprothese;
- Fig. 4 und 5: Darstellungen zur Notwendigkeit des Einkürzens der Prothesenzähne;
- Fig. 6 bis 8: Beispiele eines Prothesenzahnträgers eines erfindungsgemäßen Sets;
- Fig. 9: eine schematisierte Darstellung zur erfindungsgemäßen Bearbeitung der in einem Prothesenzahnträger eines erfindungsgemäßen Sets angeordneten Prothesenzähne;
- Fig. 10 bis 15: schematisierte Darstellungen zur Herstellung des Prothesenzahnträgers eines erfindungsgemäßen Sets;
- Fig. 16 und 17: schematisierte Darstellungen zum Einkürzen der im Prothesenzahnträger angeordneten Prothesenzähne.

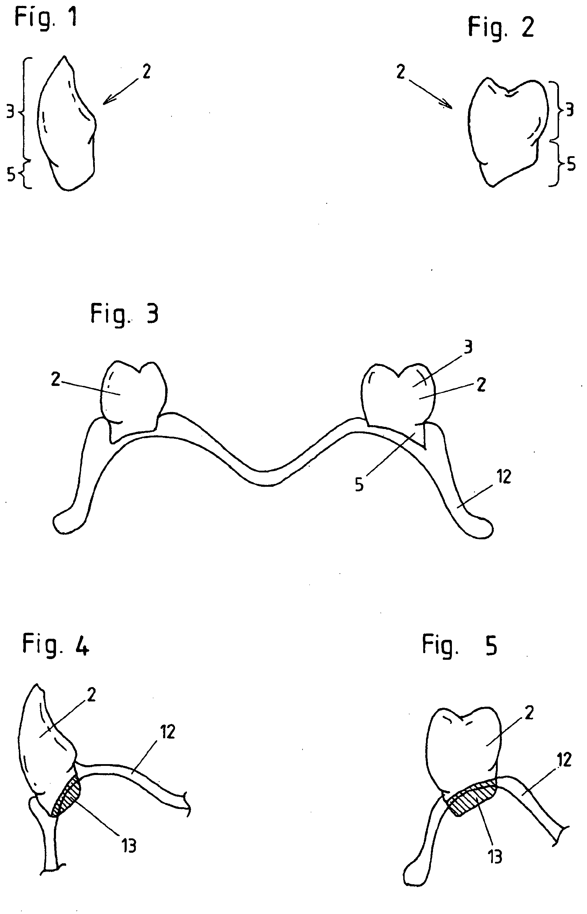

In Fig. 1 ist schematisiert ein beim Stand der Technik an sich bekannter und am Markt so auch erhältlicher Prothesenzahn 2 in Form eines Frontzahnes gezeigt. Fig. 2 zeigt beispielhaft einen Prothesenzahn 2 in Form eines Backenzahns. In beiden Figuren sind der Kronenbereich 3 und der Zahnhals- und Wurzelbereich 5 des jeweiligen Prothesenzahns 2 eingezeichnet. Fig. 3 zeigt einen Querschnitt durch eine Vollprothese. In der Prothesenbasis 12 sind in den entsprechenden Ausnehmungen die Prothesenzähne 2 mittels ihrer Zahnhals- und Wurzelbereiche 5 befestigt. Die Länge des Zahnhals- und Wurzelbereichs 5 gibt vor, wie weit der gesamte Prothesenzahn aus der Prothesenbasis 12 heraussteht. Das untere Ende des Zahnhals- und Wurzelbereichs 5 bildet mit der entsprechenden Gegenfläche der Prothesenbasis 12 einen entsprechenden Anschlag und die Abstützfläche für den Prothesenzahn 2. Bei den Prothesenzähnen 2 handelt es sich um vorgefertigte Produkte, welche am Markt in einer vorkonfektionierten Form und Größe angeboten werden. Die Prothesenbasis hingegen ist individuell an den Kiefer des jeweiligen Patienten anzupassen. Um auch die Länge des jeweiligen Prothesenzahnes 2 an die individuellen Bedürfnisse des Patienten anpassen zu können, ist es, wie eingangs bereits erläutert, notwendig, den jeweiligen Prothesenzahn an den in der Prothesenbasis zur Verfügung stehenden Platz in seiner Länge anzupassen. Hierzu muss ein Prothesenzahn 2 gegebenenfalls in seiner Länge gekürzt werden. Dies hat durch entsprechende Entfernung eines Teilbereichs 13 des Zahnhals- und Wurzelbereichs 5 zu erfolgen, wie dies schematisiert in den Fig. 4 und 5 dargestellt ist.

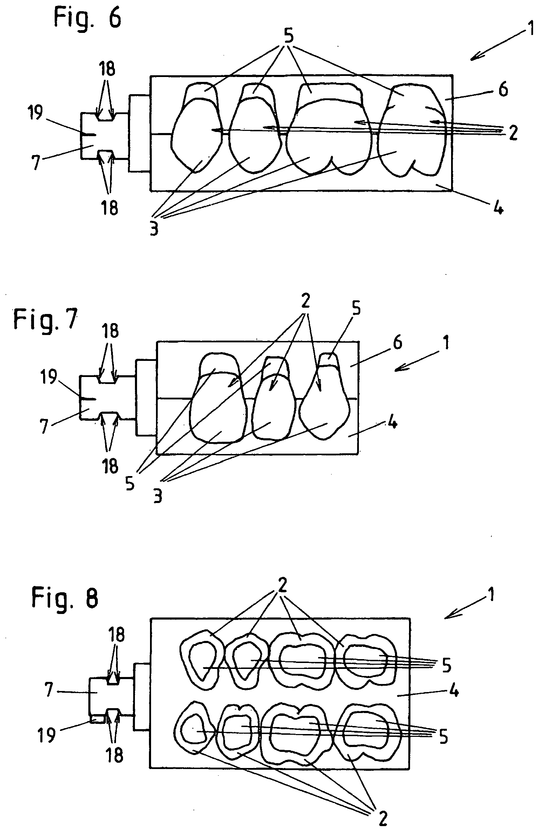

Erfindungsgemäß ist zur Automatisierung dieses Einkürzvorgangs die Verwendung von Prothesenzahnträgern 1 vorgesehen, welche beispielhaft in den Fig. 6 bis 8 dargestellt sind. Die Prothesenzahnträger 1 können einen aber auch mehrere Prothesenzähne 2 beinhalten. Bevorzugt umfasst ein Prothesenzahnträger vier oder acht Backenzähne oder drei oder sechs Frontzähne. In Fig. 6 ist eine Seitenansicht auf einen Prothesenzahnträger 1 mit insgesamt acht als Backenzahnersatz vorgesehenen Prothesenzähnen 2 dargestellt. In der Seitenansicht gemäß Fig. 6 sind nur vier dieser Prothesenzähne 2 zu sehen. Fig. 8 zeigt eine Draufsicht auf die Zahnhals- und Wurzelbereiche 5 dieser Prothesenzähne 2. In Fig. 8 sind daher auch alle acht Prothesenzähne 2 zu sehen.

Fig. 7 zeigt eine zu Fig. 6 entsprechende Seitenansicht, in der drei als Frontzahnersatz vorgesehene Prothesenzähne 2 angeordnet sind. Die Prothesenzähne 2 sind jeweils mit ihren Kronenbereichen 3 in die Trägerschicht 4 des jeweiligen Prothesenzahnträgers 1 eingebettet und dort festgehalten. Die Abdeckschicht 6, welche die Zahnhals- und Wurzelbereiche 5 einbettet ist optional. In den Fig. 6 und 7 ist sie vorhanden, sie kann aber auch weggelassen sein, sodass die Zahnhals- und Wurzelbereiche 5 dann frei von außen einsehbar über die Trägerschicht 4 überstehen. Der Vollständigkeit halber wird darauf hingewiesen, dass die Trägerschicht 4 und die Abdeckschicht 6 in den Fig. transparent dargestellt sind, damit man die Prothesenzähne 2 sieht. In Realität muss diese Transparenz natürlich nicht gegeben sein. Die Prothesenzähne können in der Trägerschicht 4 und der gegebenenfalls vorhandenen Abdeckschicht 6 auch nicht von außen sichtbar verborgen sein.

An den in den Fig. 6 bis 8 dargestellten Prothesenzahnträgern 1 ist jeweils ein Fräsmaschinenadapter 7 befestigt, der dem Einspannen des Prothesenzahnträgers 1 in einer an sich bekannten Fräseinrichtung 10 dient. Die Fräsmaschinenadapter 7 sind günstigerweise so in ihrer Form ausgebildet bzw. indiziert, dass sie ausschließlich in einer einzigen Position in einer entsprechenden Aufnahme der Fräsmaschine befestigt werden können. In den Fig. 6 bis 8 ist dies durch die für eine formschlüssige Befestigung in der Fräseinrichtung 10 vorgesehene Schulterflächen 18 und durch die Indexnase 19 erreicht.

Im Sinne des erfindungsgemäßen Sets sind die Positionen und die Formen der Prothesenzähne 2 im Prothesenzahnträger 1 bekannt und in Form eines Datensatzes 9 abgespeichert. Hierdurch wird es der mittels der Datenverarbeitungseinrichtung 11 gesteuerten Fräseinrichtung 10 möglich, die Zahnhals- und Wurzelbereiche 5 der jeweiligen Prothesenzähne 2 in Abhängigkeit der jeweiligen Anforderungen, an die individuell auszugestaltende Voll- oder Teilprothese angepasst, zu bearbeiten bzw. einzukürzen.

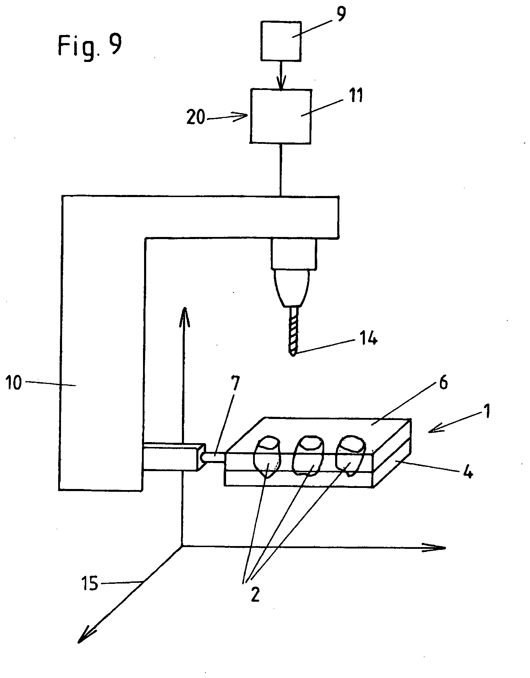

Fig. 9 zeigt schematisiert eine mittels der Datenverarbeitungseinrichtung 11 kontrollierte und angesteuerte Fräseinrichtung 10. Geeignete Fräseinrichtungen 10 sind z.B. als CNC-Fräsmaschinen beim Stand der Technik bekannt. Der schematisiert in Fig. 9 dargestellte Prothesenzahnträger 1 ist mittels seines Fräsmaschinenadapters 7 in der Fräseinrichtung 10 eingespannt. Für das Bearbeiten bzw. Abfräsen der Zahnhals- und Wurzelbereiche 5 sowie der gegebenenfalls vorhandenen Abdeckschicht 6 des Prothesenzahnträgers 1 ist ein schematisiert dargestellter Fräskopf 14 vorgesehen. Fräskopf 14 und Prothesenzahnträger 1 können in an sich bekannter Weise so relativ zueinander positioniert und verstellt werden, dass der Fräskopf 14 alle gewünschten Bereiche des Prothesenzahnträgers 1 und insbesondere der Zahnhals- und Wurzelbereiche 5 der im Prothesenzahnträger 1 angeordneten Prothesenzähne 2 in der gewünschten Art bearbeiten bzw. abfräsen kann. Gesteuert wird der Fräsvorgang der Fräseinrichtung mittels der Datenverarbeitungseinrichtung 11. Die Position und Form der Prothesenzähne 2 im jeweiligen Prothesenzahnträger 11 enthält der Datensatz 9, welcher über eine geeignete Art und Weise von der Datenverarbeitungseinrichtung 11 eingelesen wird. Über an sich bekannte Möglichkeiten werden der Datenverarbeitungseinrichtung 11 zusätzlich noch die Sollwerte 20 zugeführt, welche vorgeben, um welches Maß der oder die Prothesenzähne 2 bearbeitet bzw. eingekürzt werden muss bzw. müssen. Mit einer in Fig. 9 schematisiert dargestellten Anordnung kann das eingangs erwähnte Verfahren zur Bearbeitung des oder der Prothesenzähne durchgeführt werden. Fig. 9 stellt schematisiert ein gemeinsames Koordinatensystem 15 dar, auf dessen Basis die Datenverarbeitungseinrichtung 11 arbeitet und auf dessen Basis auch die Informationen zur Position und Form des Prothesenzahns 2 bzw. der Prothesenzähne 2 im Prothesenzahnträger 1 im Datensatz 9 abgespeichert sind. Mit dem Fräskopf 14 können nicht nur die Zahnhals- und Wurzelbereiche 5 und die gegebenenfalls vorhandene Abdeckschicht 6 bearbeitet bzw. abgefräst werden. Mittels des Fräskopfes 14 kann bevorzugt auch eine entsprechende Sollbruchstelle in der Trägerschicht 4 eingefräst werden, um nach Fertigstellung der Bearbeitung der Zahnhals- und Wurzelbereiche 5 und gegebenenfalls der Zahnkronenbereiche die Prothesenzähne 2 aus dem Prothesenzahnträger 1 entnehmen zu können. Solche Sollbruchstellen können aber natürlich auch bereits vorab vom Hersteller im Prothesenzahnträger 1 eingearbeitet werden.

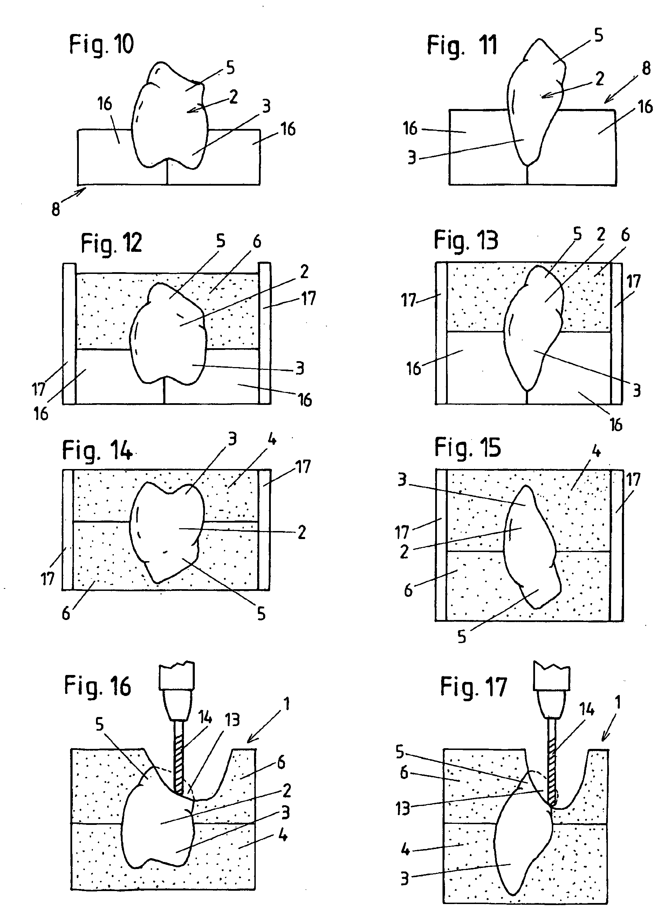

Die Fig. 10 bis 15 veranschaulichen beispielhaft ein Verfahren zur Herstellung eines Prothesenzahnträgers 1. Die Fig. 10, 12 und 14 zeigen einzelne Schritte am Beispiel eines als Backenzahn ausgebildeten Prothesenzahns 2, die Fig. 11, 13 und 15 zeigen dasselbe beispielhaft anhand eines als Frontzahn vorgesehenen Prothesenzahns 2. Die Prothesenzähne 2 sind, wie eingangs bereits erläutert, in der dargestellten Form am Markt erhältlich. Sie werden mittels einer geeigneten Hilfshaltevorrichtung in eine definierte Position gebracht und dort gehalten. Dies ist in Fig. 10 und 11 dargestellt. In beiden Figuren bestehen die Hilfshaltevorrichtungen 8 aus jeweils zwei Haltebacken 16, welche auseinander und aufeinander zu gefahren werden können. In den Fig. 10 und 11 ist der jeweilige Prothesenzahn 2 bereits entsprechend zwischen den Haltebacken 16 in der jeweiligen Hilfshaltevorrichtung 8 positioniert und gehalten, wodurch die Position des jeweiligen Prothesenzahns 2 im zugrundeliegenden Koordinatensystem 15 bekannt ist. Die Hilfshaltevorrichtungen 8 können unterschiedlich viele Aufnahmen für unterschiedlich viele Prothesenzähne 2 aufweisen. Die Anzahl ist günstigerweise jeweils auf den herzustellenden Prothesenzahnträger und die in ihm vorgesehene Anzahl von Prothesenzähnen 2 abzustimmen.

Der Vollständigkeit halber sei darauf hingewiesen, dass bei einer entsprechenden Ausformung der Prothesenzähne 2 die Hilfshaltevorrichtungen 8 nicht unbedingt zwei- bzw. mehrteilig ausgeführt sein müssen. Im Allgemeinen ist dies aber günstig. Beispielsweise können die Zähne in einteiligen Formen auch mittels Ansaugmechanismen in der Form fixiert werden.

In den Fig. 12 und 13 erfolgt nun der nächste Verfahrensschritt. Zunächst wird der Prothesenzahn 2 samt Hilfshaltevorrichtung 8 mittels des Mantels 17 ummantelt. Anschließend wird die Abdeckschicht 6 gegossen oder umspritzt, womit ein über die Haltevorrichtung 8 hervorstehender Teilbereich der Prothesenzähne 2 in die Abdeckschicht 6 eingebettet wird. In der Abdeckschicht 6 sollte sich vorzugsweise der gesamte Zahnhals- und Wurzelbereich 5 des Prothesenzahns bzw. der Prothesenzähne 2 befinden. In der Abdeckschicht 6 können sich aber auch Teile des Kronenbereichs 3 befinden. Der Kronenbereich 3 muss letztendlich nur so weit in der Trägerschicht 4 eingebettet sein, dass der jeweilige Prothesenzahn 2 bei der Bearbeitung mittels Fräskopf 14 ausreichend festgehalten ist. Nach Aushärten der Abdeckschicht 6, welche vorzugweise aus einem geeigneten fräsbaren Kunststoff oder Wachs besteht, kann die Hilfshaltevorrichtung 8 entfernt werden. Die Prothesenzähne 2 sind dann in der Abdeckschicht 6 positionstreu gehalten. Nach Entfernen der Hilfshaltevorrichtung 8 kann dann die Trägerschicht 4 gegossen oder umspritzt werden, sodass der Kronenbereich des Prothesenzahns 2 bzw. der Prothesenzähne 2 in der Trägerschicht 4 eingebettet ist. Nach gegebenenfalls notwendigem Aushärten des Materials der Trägerschicht 4 kann der Mantel 17 entfernt werden. Anschließend wird der Fräsmaschinenadapter 7 noch am Prothesenzahnträger 1 angebracht. In den Fig. 16 und 17 ist dann noch schematisiert dargestellt, wie der so fertiggestellte Prothesenzahnträger 1 nach dem Einspannen in der Fräseinrichtung 10 mittels des Fräskopfes 14 bearbeitet wird, indem im benötigten Umfang die Abdeckschicht 6 und der zu entfernende Teilbereich 13 des Zahnhals- und Wurzelbereichs 5 nach den Vorgaben der Datenverarbeitungseinrichtung 11 abgefräst werden.

Die Fig. 10 bis 17 beziehen sich auf ein Ausführungsbeispiel bei dem der Prothesenzahnträger 1 sowohl eine Trägerschicht 4 als auch eine Abdeckschicht 6 aufweisen.

Wie bereits eingangs erläutert, ist die Abdeckschicht 6 aber nicht unbedingt zwingend notwendig. Um einen Prothesenzahnträger 1 ohne Abdeckschicht 6 herzustellen, kann das anhand der Fig. 10 bis 15 erläuterte Herstellungsverfahren dahingehend abgewandelt werden, dass die vorkonfektionierten Prothesenzähne 2 nicht mit ihrem Kronenbereich 3 sondern mit ihrem Zahnhals- und Wurzelbereich 5 in einer geeignet ausgeformten, die Positionsvorgabe beinhaltenden Hilfshaltevorrichtungen 8 positioniert werden. Anschließend kann ein entsprechender Mantel 17 an der Hilfshaltevorrichtung 8 angebracht werden. Danach wird dann direkt die Trägerschicht 4 gegossen oder umspritzt und damit die frei über die Hilfshaltevorrichtung 8 überstehenden Bereiche des Kronenbereichs 3 der Prothesenzähne 2 eingebettet. Nach Entfernen des Mantels 17 und der Hilfshaltevorrichtung 8 ist dann eine Trägerschicht 4 mit darin angeordneten Prothesenzähnen 2 hergestellt, bei der die Zahnhals- und Wurzelbereiche 5 über die Trägerschicht 4 frei überstehen und entsprechend mittels eines geeigneten Fräskopfes 14 in einer Fräseinrichtung 10 bearbeitet werden können.

### Legende

### zu den Hinweisziffern:

- 1: Prothesenzahnträger
- 2: Prothesenzahn
- 3: Kronenbereich
- 4: Trägerschicht
- 5: Zahnhals- und Wurzelbereich
- 6: Abdeckschicht
- 7: Fräsmaschinenadapter
- 8: Hilfshaltevorrichtung
- 9: Datensatz
- 10: Fräseinrichtung
- 11: Datenverarbeitungseinrichtung
- 12: Prothesenbasis
- 13: zu entfernender Teilbereich
- 14: Fräskopf
- 15: Koordinatensystem
- 16: Haltebacken
- 17: Mantel
- 18: Schulterfläche
- 19: Indexnase
- 20: Sollwerte

## Patentansprüche

1. Set aus einem Prothesenzahnträger (1) mit zumindest einem Prothesenzahn (2), insbesondere mit mehreren Prothesenzähnen (2), und einem Datensatz (9), wobei der Datensatz (9) die Information über die Position des Prothesenzahns (2) bzw. der Prothesenzähne (2) im und/oder am Prothesenzahnträger (1) und die Information über die Form des Prothesenzahns (2) bzw. der Prothesenzähne (2) beinhaltet, **dadurch gekennzeichnet, dass** ein Kronenbereich (3) des Prothesenzahns (2) bzw. der Prothesenzähne (2) zumindest bereichsweise in eine Trägerschicht (4) eingegossen oder umspritzt ist und an der Trägerschicht (4) ein Fräsmaschinenadapter (7) des Prothesenzahnträgers (1) zur Befestigung des Prothesenzahnträgers (1) während eines Fräsvorgangs in einer Fräseinrichtung (10) befestigt ist.

2. Set nach Anspruch 1, **dadurch gekennzeichnet, dass** ein Zahnhals- und Wurzelbereich (5) des Prothesenzahnes (2) bzw. der Prothesenzähne (2) zumindest bereichsweise, vorzugsweise vollständig, frei einsehbar über die Trägerschicht (4) übersteht.

3. Set nach Anspruch 1, **dadurch gekennzeichnet, dass** ein Zahnhals- und Wurzelbereich (5) des Prothesenzahnes (2) bzw. der Prothesenzähne (2) zumindest bereichsweise, vorzugsweise vollständig, in eine Abdeckschicht (6) des Prothesenzahnträgers (1) eingebettet, vorzugsweise eingegossen, ist.

4. Verfahren zur Bearbeitung zumindest eines Prothesenzahns (2), vorzugsweise mehrerer Prothesenzähne (2), eines Sets nach einem der Ansprüche 1 bis 3, wobei der Prothesenzahnträger (1) des Sets mittels seines Fräsmaschinenadapters (7) in eine Fräseinrichtung (10) eingespannt wird und der Datensatz (9) des Sets in eine Datenverarbeitungseinrichtung (11) eingelesen wird und die Fräseinrichtung (10) von der Datenverarbeitungseinrichtung (11) zum Bearbeiten des Prothesenzahns (2) oder der Prothesenzähne (2) unter Berücksichtigung des Datensatzes (9) angesteuert wird.

5. Verfahren nach Anspruch 4, **dadurch gekennzeichnet, dass** der Datensatz (9) im oder am Prothesenzahnträger (1), vorzugsweise im oder am Fräsmaschinenadapter (7), gespeichert wird und beim oder nach dem Einspannen des Prothesenzahnträgers (1) in die Fräseinrichtung (10), vorzugsweise automatisch, von der Datenverarbeitungseinrichtung (11) eingelesen wird.

6. Verfahren zur Herstellung eines Sets nach einem der Ansprüche 2 oder 3, **dadurch gekennzeichnet, dass** der Prothesenzahn (2) bzw. die Prothesenzähne (2) mit dem Zahnhals- und Wurzelbereich (5) in einer Hilfshaltevorrichtung (8) positioniert wird bzw. werden und dann der Kronenbereich (3) des Prothesenzahns (2) bzw. der Prothesenzähne (2) zumindest bereichsweise, vorzugsweise vollständig, in der Trägerschicht (4) eingegossen oder umspritzt wird bzw. werden.

7. Verfahren zur Herstellung eines Sets nach Anspruch 3, **dadurch gekennzeichnet, dass** der Prothesenzahn (2) bzw. die Prothesenzähne (2) mit dem Kronenbereich (3) in einer Hilfshaltevorrichtung (8) positioniert wird bzw. werden und dann der Zahnhals- und Wurzelbereich (5) des Prothesenzahnes (2) bzw. der Prothesenzähne (2) zumindest bereichsweise, vorzugsweise vollständig, in der Abdeckschicht (6) des Prothesenzahnträgers (1) eingebettet, vorzugsweise eingegossen, wird bzw. werden und daran anschließend der Kronenbereich (3) des Prothesenzahns (2) bzw. der Prothesenzähne (2) zumindest bereichsweise, vorzugsweise vollständig, in die Trägerschicht (4) eingegossen oder umspritzt wird bzw. werden.

## Claims

1. A set consisting of a prosthetic tooth support (1) with at least one prosthetic tooth (2), in particular with a plurality of prosthetic teeth (2), and with a data set (9), wherein the data set (9) contains the information about the position of the prosthetic tooth (2) or the prosthetic teeth (2) in and/or on the prosthetic tooth support (1) and the information about the shape of the prosthetic tooth (2) or the prosthetic teeth (2), **characterised in that** a crown region (3) of the prosthetic tooth (2) or of the prosthetic teeth (2) is cast or encapsulated at least in regions in a support layer (4), and a milling-machine adapter (7) of the prosthetic tooth support (1) is fastened on the support layer (4) in order to fasten the prosthetic tooth support (1) during a milling operation in a milling means (10).

2. A set according to Claim 1, **characterised in that** a tooth neck and root region (5) of the prosthetic tooth (2) or of the prosthetic teeth (2) projects at least in regions, preferably completely, freely visibly over the support layer (4).

3. A set according to Claim 1, **characterised in that** a tooth neck and root region (5) of the prosthetic tooth (2) or of the prosthetic teeth (2) at least in regions, preferably completely, is embedded, preferably cast, in a cover layer (6) of the prosthetic tooth support (1).

4. A method for machining at least one prosthetic tooth (2), preferably a plurality of prosthetic teeth (2), of a set according to one of Claims 1 to 3, wherein the prosthetic tooth support (1) of the set, by means of its milling-machine adapter (7), is clamped in a milling means (10) and the data set (9) of the set is read into a data-processing means (11) and the milling means (10) is controlled by the data-processing means (11) for machining the prosthetic tooth (2) or the prosthetic teeth (2), taking into account the data set (9).

5. A method according to Claim 4, **characterised in that** the data set (9) is stored in or on the prosthetic tooth support (1), preferably in or on the milling-machine adapter (7), and is read in upon or after the clamping of the prosthetic tooth support (1) into the milling means (10), preferably automatically, by the data-processing means (11).

6. A method for producing a set according to one of Claims 2 or 3, **characterised in that** the prosthetic tooth (2) or the prosthetic teeth (2) is or are positioned with the tooth neck and root region (5) in an auxiliary holding device (8) and then the crown region (3) of the prosthetic tooth (2) or of the prosthetic teeth (2) at least in regions, preferably completely, is or are cast or encapsulated into the support layer (4).

7. A method for producing a set according to Claim 3, **characterised in that** the prosthetic tooth (2) or the prosthetic teeth (2) is or are positioned with the crown region (3) in an auxiliary holding device (8) and then the tooth neck and root region (5) of the prosthetic tooth (2) or of the prosthetic teeth (2) at least in regions, preferably completely, is or are embedded, preferably cast, in the cover layer (6) of the prosthetic tooth support (1) and then the crown region (3) of the prosthetic tooth (2) or of the prosthetic teeth (2) at least in regions, preferably completely, is or are cast or encapsulated into the support layer (4).

## Revendications

1. Ensemble d'un support de dent prothétique (1) comportant au moins une dent prothétique (2), en particulier comportant plusieurs dents prothétiques (2), et un jeu de données (9), dans lequel le jeu de données (9) contient l'information sur la position de la dent prothétique (2) ou des dents prothétiques (2) dans et/ou sur le support de dent prothétique (1) et l'information sur la forme de la dent prothétique (2) ou des dents prothétiques (2), **caractérisé en ce qu'**une zone de couronne (3) de la dent prothétique (2) ou des dents prothétiques (2) est coulée ou recouverte par injection, au moins partiellement dans une couche de support (4) et à la couche de support (4) est fixé un adaptateur de machine de fraisage (7) du support de dent prothétique (1) pour la fixation du support de dent prothétique (1) pendant un processus de fraisage dans un équipement de fraisage (10).

2. Ensemble selon la revendication 1, **caractérisé en ce qu'**une zone de collet dentaire et de racine (5) de la dent prothétique (2) ou des dents prothétiques (2) dépasse au moins partiellement, de préférence complètement, de façon à pouvoir être librement visible au-dessus de la couche de support (4).

3. Ensemble selon la revendication 1, **caractérisé en ce qu'**une zone de collet dentaire et de racine (5) de la dent prothétique (2) ou des dents prothétiques (2) est englobée, de préférence coulée, au moins partiellement, de préférence complètement, dans une couche de recouvrement (6) du support de dent prothétique (1).

4. Procédé de traitement d'au moins une dent prothétique (2), de préférence de plusieurs dents prothétiques (2), d'un ensemble selon l'une des revendications 1 à 3, dans lequel le support de dent prothétique (1) de l'ensemble est serré au moyen de son adaptateur de machine de fraisage (7) dans un équipement de fraisage (10) et le jeu de données (9) de l'ensemble est lu dans un dispositif de traitement de données (11) et l'équipement de fraisage (10) du dispositif de traitement des données (11) pour le traitement de la dent prothétique (2) ou des dents prothétiques (2) est commandé en tenant compte du jeu de données (9).

5. Procédé selon la revendication 4, **caractérisé en ce que** le jeu de données (9) dans ou sur le support de dent prothétique (1) est enregistré, de préférence dans ou sur l'adaptateur de machine de fraisage (7), et lors du serrage ou après le serrage du support de dent prothétique (1) dans l'appareil de fraisage (10), est lu, de préférence automatiquement, par le dispositif de traitement des données (11).

6. Procédé de production d'un ensemble selon l'une des revendications 2 ou 3, **caractérisé en ce que** la dent prothétique (2) ou les dents prothétiques (2) est ou sont positionnée(s) avec la zone de collet dentaire et de racine (5) dans un dispositif de maintien auxiliaire (8) et ensuite, la zone de couronne (3) de la dent prothétique (2) ou des dents prothétiques (2) est coulée ou recouverte par injection au moins partiellement, de préférence complètement, dans la couche de support (4).

7. Procédé de production d'un ensemble selon la revendication 3, **caractérisé en ce que** la dent prothétique (2) ou les dents prothétiques (2) avec la zone de couronne (3) est ou sont positionnée(s) dans un dispositif de maintien auxiliaire (8) et ensuite, la zone de collet dentaire et de racine (5) de la dent prothétique (2) ou des dents prothétiques (2) est ou sont englobée(s), de préférence coulée(s), au moins partiellement, de préférence complètement, dans la couche de recouvrement (6) du support de dent prothétique (1) et ensuite, la zone de couronne (3) de la dent prothétique (2) ou des dents prothétiques (2) est ou sont coulée(s) ou recouverte(s) par injection au moins partiellement, de préférence complètement, dans la couche de support (4).
